# EUROPEAN PATENT SPECIFICATION

(11) **EP 3 050 957 B1**
(45) Date of publication and mention of the grant of the patent: **26.06.2019**
(21) Application number: 14848746.5
(22) Date of filing: 19.09.2014
(51) Int. Cl.: C12M 1/00, C12N 1/00, C12M 3/00

(54) **AUTOMATIC CULTURE SYSTEM AND AUTOMATIC CULTURE DEVICE**
AUTOMATISCHES KULTURSYSTEM UND AUTOMATISCHE KULTURVORRICHTUNG
SYSTÈME ET DISPOSITIF DE CULTURE AUTOMATISÉE

(30) Priority: 25.09.2013 JP 2013198921
(43) Date of publication of application: 03.08.2016
(73) Proprietor: Sinfonia Technology Co., Ltd., Tokyo 105-8564 (JP)
(72) Inventor: OZAKI, Shigenori, Nirasaki City, Yamanashi 407-0192 (JP); FUJI, Toshimitsu, Tokyo 107-6325 (JP); KINOSHITA, Yoshio, Tokyo 107-6325 (JP); TAMURA, Akitake, Tokyo 107-6325 (JP)
(74) Representative: Diehl & Partner GbR
(86) International application number: PCT/JP2014/074880
(87) International publication number: WO 2015/046068

(56) References cited:
- WO-A1-2011/001995
- WO-A1-2011/087053
- WO-A1-2012/020458
- WO-A1-2012/141055
- WO-A1-2014/155500
- JP-A- 2009 189 362
- JP-A- 2009 219 415
- JP-A- 2010 075 200
- US-A- 5 547 329
- DATABASE WPI Week 200966 Thomson Scientific, London, GB; AN 2009-P27056 XP002769184, & JP 2009 219415 A (HITACHI PLANT ENG&CONSTR CO LTD) 1 October 2009 (2009-10-01)
- DATABASE WPI Week 200720 Thomson Scientific, London, GB; AN 2007-196094 XP002769186, -& JP 2007 037422 A (OLYMPUS OPTICAL CO LTD) 15 February 2007 (2007-02-15)

## Description

### TECHNICAL FIELD

The present disclosure relates to an automatic culture system which automatically cultures cells, and an automatic culture method which automatically cultures cells using the automatic culture system.

### BACKGROUND

In recent years, research and development of regenerative medicine that artificially creates tissues and organs of interest by virtue of cell culture are underway. When performing a culture operation of cells, a cell culture facility which meets predetermined criteria, for example, a GMP (Good Manufacturing Practice), is used. In the cell culture facility, an aseptic management and an aseptic manipulation are needed. As the cell culture facility, there is known, for example, a CPC (Cell Processing Center).

In this cell culture facility, an attempt is made to automate cell culture to some extent. Japanese Patent No. 4803196 discloses an arrangement that includes a plurality of cell culture rooms, a raw material processing room, a product processing room, and a transfer robot for automatically performing the transfer of cells between the cell culture rooms, the raw material processing room and the product processing room. An automatic cell culture device is disposed in each of the cell culture rooms. Raw material cells can be automatically supplied to a selected cell culture room by the transfer robot. Cultured product cells can be automatically carried into the product processing room.

However, in Japanese Patent No. 4803196 mentioned above, it is assumed that a worker intervenes in the culture of cells. It is not assumed that the culture of cells is fully automated. Since the worker intervenes in this way, it is necessary to thoroughly perform sterilization of the worker. In a work site where cells assumed to be finally returned to a human body or the like, such as iPS cells or the like, are handled, sterilization of a worker creates a very uneasy situation. Equipment costs, maintenance costs, etc. are also high.
JP 2009 219415 (A) discloses an automatic cell culture facility characterized by disposing a plurality of cell culture chambers in parallel, disposing a cell conveying robot chamber having a robot for automatically conveying cells between each culture chamber and a raw material-treating chamber or a product-treating chamber, and forming a material-setting chamber for setting a culture medium to a culture apparatus for automatically culturing the cells in the cell culture chambers in a state sharing the material-setting chamber by the cell culture chambers. A dressing chamber and an undressing chamber for going in or out the material-setting chamber are disposed in the material-setting chamber for sharing. Products are transferred to the product-treating chamber through a pass box connected to a ceiling conveying route between the product-treating chamber and an inspection chamber or product-shipping chamber disposed separately. A pressure gradient for positively pressuring the product-treating chamber is imparted between chambers connected to each other through the ceiling conveying route.

In view of these points, the present disclosure provides some embodiments of an automatic culture system and an automatic culture method which are capable of completely automatically culturing cells and which do not necessarily need to maintain high cleanliness in a cell culture area.

### SUMMARY

The above object is solved by the combination of features of the independent claims. Preferred embodiments are defined in the dependent claims.

An automatic culture system according to the present disclosure includes: a transfer part configured to transfer a first airtight container which accommodates cells in a sealed state; and an automatic culture device configured to receive the first airtight container transferred by the transfer part, take out the cells from the first airtight container and culture the taken-out cells, wherein the automatic culture device is configured to automatically perform replacement of a culture medium.

In the system according to the present disclosure, the automatic culture device may be configured to automatically perform subculture of the cells.

In the system according to the present disclosure, the first airtight container may be transferred by the transfer part in a state in which a second airtight container that accommodates the cells is accommodated within the first airtight container.

In the system according to the present disclosure, the first airtight container may be transferred by the transfer part in a state in which a plurality of second airtight containers is accommodated within the first airtight container.

In the system according to the present disclosure, the automatic culture device may be configured to receive the first airtight container which accommodates the second airtight container and which is transferred by the transfer part, take out the second airtight container from the first airtight container, and automatically culture the cells in a state in which the cells are accommodated in the taken-out second airtight container.

The system according to the present disclosure may further include: a sterilizing device configured to sterilize an interior of the first airtight container which accommodates the cells.

In the system according to the present disclosure, the sterilizing device may be configured to sterilize the interior of the first airtight container which accommodates the cells, by supplying a sterilizing gas into the first airtight container.

In the system according to the present disclosure, the automatic culture device may include a discharge part configured to discharge at least a waste liquid.

In the system according to the present disclosure, at least one of a temperature, a humidity and a gas concentration within the first airtight container may be adjustable.

The system according to the present disclosure may further include: a temporary storage part configured to temporarily store the first airtight container, wherein at least one of a temperature, a humidity and a gas concentration within the temporary storage part may be adjustable.

In the system according to the present disclosure, the automatic culture device may include a cell loading part configured to load the first airtight container which accommodates the cells and a material loading part configured to load materials.

In the system according to the present disclosure, the automatic culture device may include a plurality of automatic culture devices, the system may further include a plurality of accommodation rooms configured to accommodate the automatic culture devices, and the automatic culture devices may be respectively accommodated in the corresponding accommodation rooms in a one-to-one relationship.

An automatic culture method according to the present disclosure includes: transferring, by a transfer part, a first airtight container which accommodates cells in a sealed state; receiving, by an automatic culture device, the first airtight container transferred by the transfer part and taking out the cells from the first airtight container; and culturing, by the automatic culture device, the taken-out cells, wherein the automatic culture device is configured to automatically adjust at least one of a temperature, a humidity and a gas concentration within the automatic culture device and to automatically perform replacement of a culture medium.

According to the present disclosure, it is possible to automatically perform the culture of cells.

### BRIEF DESCRIPTION OF THE DRAWINGS

**FIG. 1** is a schematic top plan view illustrating the configuration of an automatic culture system according to a first embodiment of the present disclosure.
**FIG. 2** is a control block diagram illustrating a control of the automatic culture system according to the first embodiment of the present disclosure.
**FIG. 3A** is a front view illustrating a transfer part used in the first embodiment of the present disclosure, and **FIG. 3B** is a top plan view illustrating one example of a second airtight container used in the first embodiment of the present disclosure.
**FIG. 4** is a view corresponding to **FIG. 1** and is a view for explaining respective areas in the automatic culture system according to the first embodiment of the present disclosure.
**FIG. 5** is a front view illustrating a loading part of an iPS-cell automatic culture device used in the first embodiment of the present disclosure.
**FIG. 6** is a front view illustrating a loading part of a differentiated-cell automatic culture device used in the first embodiment of the present disclosure.
**FIG. 7** is a schematic top plan view illustrating a case where an isolation member used in the first embodiment of the present disclosure is a double-entry door.
**FIG. 8** is a schematic top plan view illustrating a case where the isolation member used in the first embodiment of the present disclosure is an air shower.
**FIG. 9** is a schematic top plan view illustrating a case where the isolation member used in the first embodiment of the present disclosure is a single-entry door.
**FIG. 10** is a schematic top plan view illustrating the configuration of an automatic culture system according to a second embodiment of the present disclosure, which includes a culture area where iPS cells are cultured and which does not include a differentiation area where iPS cells are differentiated.
**FIG. 11** is a schematic top plan view illustrating the configuration of an automatic culture system according to a second embodiment of the present disclosure, which includes both a culture area where iPS cells are cultured and a differentiation area where iPS cells are differentiated.

### DETAILED DESCRIPTION

### First Embodiment

### <<Configuration>>

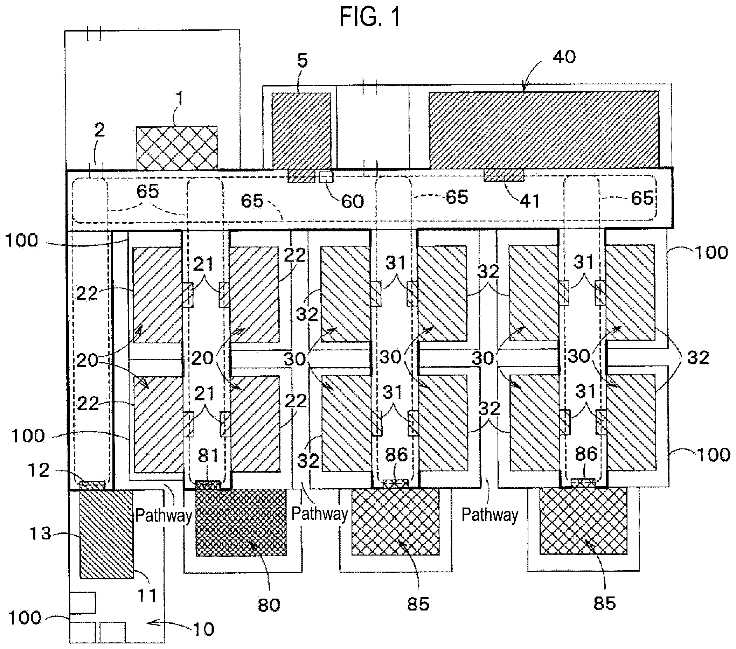

A first embodiment of an automatic culture system and an automatic culture method according to the present disclosure will now be described in detail with reference to the accompanying drawings. **FIGS. 1** to **9** are views for explaining the first embodiment of the present disclosure.

The automatic culture system of the present embodiment is a system for automatically culturing different cells including pluripotent stem cells such as iPS cells, ES cells or the like, cartilage cells such as bone marrow stromal cells (MSCs) or the like, dendritic cells, and the like. In the present embodiment, an aspect using iPS cells will be mainly described hereinafter. However, it is to be understood that this is nothing more than one example.

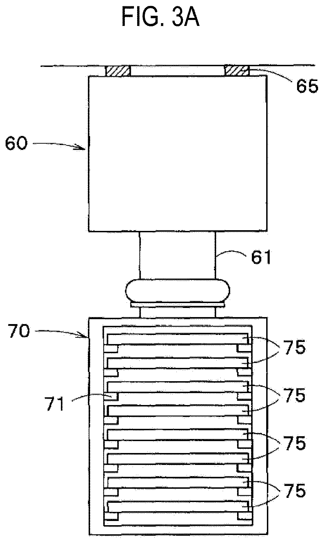

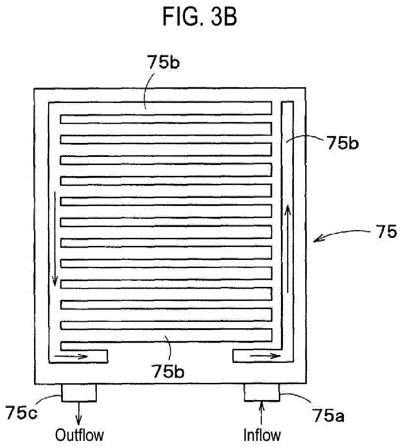

As illustrated in **FIG. 1****,** the automatic culture system according to the present embodiment includes a raw material storage device 10 which stores cells, a transfer part 60 which transfers a first airtight container 70 (*see* **FIGS. 3A** and **3B**) that accommodates cells in a sealed state, and automatic culture devices 20 and 30 which receive the first airtight container 70 transferred by the transfer part 60, take out the cells from the first airtight container 70 and culture the taken-out cells therein.

In the present embodiment, as described above, an aspect using iPS cells is described. Thus, the raw material storage device 10 includes an iPS cell establishing device 11 which establishes iPS cells. In addition, the raw material storage device 10 includes a unit thermostatic bath, a centrifuge, an automatic blood cell counting device, an automatic magnetic cell separator, a flow cytometer, a gene introduction device, and so forth.

The automatic culture devices 20 and 30 of the present embodiment include a plurality of (four, in the aspect illustrated in **FIG. 1**) iPS-cell automatic culture devices 20 which automatically culture iPS cells and a plurality of (eight, in the aspect illustrated in **FIG. 1**) differentiated-cell automatic culture devices 30 which automatically culture differentiated cells differentiated from the iPS cells. In the present embodiment, when merely saying "automatic culture devices", it refers to the iPS-cell automatic culture devices 20, the differentiated-cell automatic culture devices 30, or both of the iPS-cell automatic culture devices 20 and the differentiated-cell automatic culture devices 30. Furthermore, in the present embodiment, when merely saying "cells", it refers to raw material cells, such as somatic cells which become the source of iPS cells or the like, iPS cells, differentiated cells, or two or all of the raw material cells, the iPS cells and the differentiated cells.

In the present embodiment, in addition to the first airtight container 70, there are employed second airtight containers 75 which accommodate cells *(see* **FIGS. 3A** and **3B**). As one example, a plurality of racks 71 for mounting a plurality of (eight, in **FIG. 3A**) second airtight containers 75 is installed in the first airtight container 70. The second airtight containers 75 are disposed in the respective racks 71. In a state in which the second airtight containers 75 are accommodated within the first airtight container 70, the first airtight container 70 is transferred by the transfer part 60 in an isolated area which will be described later. The second airtight containers 75 may accommodate not only the cells but also materials such as a liquid culture medium, a chemical and the like. Incidentally, examples of the second airtight containers 75 may include a closed culture plate illustrated in **FIG. 3B****.** In the closed culture plate illustrated in **FIG. 3B****,** a liquid such as a liquid culture medium or the like flows inward from an inlet 75a, passes through a path 75b and flows out from an outlet 75c.

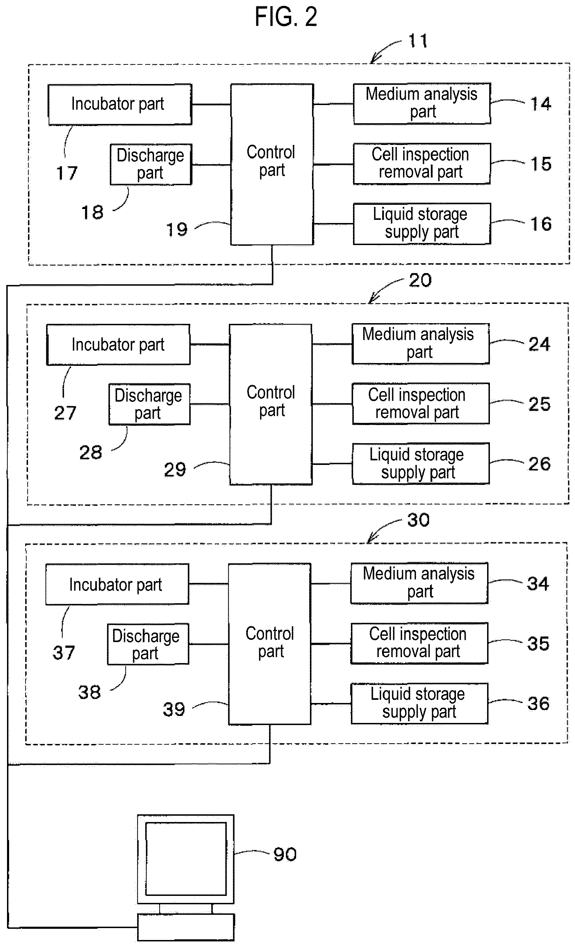

As illustrated in **FIG. 2****,** the iPS-cell automatic culture device 20 includes a housing 22 (*see* **FIG. 1**), a medium analysis part 24 which analyzes liquid culture medium components that vary with the culture of iPS cells, a cell inspection removal part 25 which inspects the iPS cells and performs removal of the iPS cells having a bad state, a liquid storage supply part 26 which stores and supplies a liquid including a liquid culture medium or a proteolytic enzyme, an incubator part 27 which holds the second airtight container 75 and automatically adjusts one or all of a temperature, a humidity and a gas concentration, and a discharge part 28 for discharging downward from the housing 22 a waste liquid including a used liquid culture medium, a used cleaning liquid, a used reagent or the like used within the iPS-cell automatic culture device 20. Incidentally, in the case where the second airtight container 75 is used as in the present embodiment, the humidity within the incubator part 27 may not be particularly managed. It is therefore possible to simplify the management of a cell culture environment. By employing the second airtight container 75, there is no fear that contamination from the air occurs. Furthermore, the transfer becomes easy.

The liquid storage supply part 26 described above appropriately supplies a liquid culture medium from the inlet 75a into the second airtight container 75, thereby automatically replacing an old liquid culture medium existing within the second airtight container 75 with a new liquid culture medium. Based on the information of the iPS cells acquired, the cell inspection removal part 25 selectively peels off defective iPS cells from an ECM (Extracellular Matrix) provided on the bottom surface of the second airtight container 75. Thereafter, a liquid culture medium is supplied from the inlet 75a into the second airtight container 75, whereby floating defective iPS cells are pushed out from the second airtight container 75. Furthermore, the liquid storage supply part 26 appropriately supplies a proteolytic enzyme from the inlet 75a into the second airtight container 75, thereby peeling off the iPS cells from the ECM provided on the bottom surface of the second airtight container 75. Thereafter, a liquid culture medium is supplied from the inlet 75a into the second airtight container 75, whereby floating iPS cells are pushed out from the second airtight container 75. The iPS cells thus pushed out are diluted into a suspension and are then accommodated (seeded) within a plurality of other second airtight containers 75. In this way, the iPS-cell automatic culture device 20 automatically performs the subculture of the iPS cells. As the method of selectively peeling off the iPS cells existing within the second airtight container 75, it may be possible to use a method of irradiating ultrasonic waves or light on the iPS cells or a method of applying a physical force from the outside of the second airtight container 75. When using these methods, a proteolytic enzyme may be used in combination.

The internal temperature of the iPS-cell automatic culture device 20 is adjusted by the incubator part 27 so that the internal temperature becomes, for example, about 37 degrees C. Furthermore, the gas concentration within the iPS-cell automatic culture device 20 is adjusted by appropriately adding CO₂ to the air using the incubator part 27. If necessary, the humidity may be adjusted by the incubator part 27 so as to become about 100%.

As illustrated in **FIG. 2****,** the differentiated-cell automatic culture device 30 includes a housing 32 (*see* **FIG. 1**), a medium analysis part 34 which analyzes liquid culture medium components that vary with the culture of differentiated cells, a cell inspection removal part 35 which inspects the differentiated cells and performs removal of the differentiated cells having a bad state, a liquid storage supply part 36 which stores and supplies a liquid including a liquid culture medium or a proteolytic enzyme, an incubator part 37 which holds the second airtight container 75 and automatically adjusts one or all of a temperature, a humidity and a gas concentration, and a discharge part 38 for discharging downward from the housing 32 a waste liquid including a used liquid culture medium, a used cleaning liquid, a used reagent or the like used within the differentiated-cell automatic culture device 30. In the case where the second airtight container 75 is used as described above, the humidity within the incubator part 37 may not be particularly managed. It is therefore possible to simplify the management of a cell culture environment. In some cases, the iPS cells cultured in the iPS-cell automatic culture device 20 may be carried into the differentiated-cell automatic culture device 30. In other cases, the iPS cells externally cultured and transferred in a frozen state may be thawed and then carried into the differentiated-cell automatic culture device 30.

The liquid storage supply part 36 described above appropriately supplies a liquid culture medium from the inlet 75a into the second airtight container 75, thereby automatically replacing an old liquid culture medium existing within the second airtight container 75 with a new liquid culture medium. Based on the information of the differentiated cells acquired, the cell inspection removal part 35 selectively peels off defective differentiated cells from an ECM (Extracellular Matrix) provided on the bottom surface of the second airtight container 75. Thereafter, a liquid culture medium is supplied from the inlet 75a into the second airtight container 75, whereby floating defective differentiated cells are pushed out from the second airtight container 75. Furthermore, the liquid storage supply part 36 appropriately supplies a proteolytic enzyme from the inlet 75a into the second airtight container 75, thereby peeling off the differentiated cells from the ECM provided on the bottom surface of the second airtight container 75. Thereafter, a liquid culture medium is supplied from the inlet 75a into the second airtight container 75, whereby floating differentiated cells are pushed out from the second airtight container 75. The differentiated cells thus pushed out are diluted into a suspension and are then accommodated (seeded) within a plurality of other second airtight containers 75. In this way, the differentiated-cell automatic culture device 30 automatically performs the subculture of the differentiated cells. As the method of selectively peeling off the differentiated cells existing within the second airtight container 75, it may be possible to use a method of irradiating ultrasonic waves or light on the differentiated cells or a method of applying a physical force from the outside of the second airtight container 75. When using these methods, a proteolytic enzyme may be used in combination.

The internal temperature of the differentiated-cell automatic culture device 30 is adjusted by the incubator part 37 so that the internal temperature becomes, for example, about 37 degrees C. Furthermore, the gas concentration within the differentiated-cell automatic culture device 30 is adjusted by appropriately adding CO₂ to the air using the incubator part 37. Moreover, the liquid storage supply part 36 of the differentiated-cell automatic culture device 30 may supply a liquid culture medium including a differentiation inducing factor when inducing differentiation. If necessary, the humidity may be adjusted by the incubator part 37 so as to become about 100%. In addition, the aforementioned process may be appropriately changed depending on the kind of differentiated cells. In the case where the shape of the differentiated cells greatly differs from the shape of the iPS cells, a closed culture plate (second airtight container) differing in shape from the culture plate (namely, the second airtight container 75) used when culturing the iPS cells is used.

As illustrated in **FIG. 2****,** the iPS-cell automatic culture device 20 includes a control part 29 connected to the medium analysis part 24, the cell inspection removal part 25, the liquid storage supply part 26, the incubator part 27 and the discharge part 28 so as to make communication therewith and configured to control them. Furthermore, the differentiated-cell automatic culture device 30 includes a control part 39 connected to the medium analysis part 34, the cell inspection removal part 35, the liquid storage supply part 36, the incubator part 37 and the discharge part 38 so as to make communication therewith and configured to control them.

As illustrated in **FIG. 2****,** the iPS cell establishing device 11 is similar in configuration to the iPS-cell automatic culture device 20 and the differentiated-cell automatic culture device 30. That is to say, the iPS cell establishing device 11 includes a housing 13 (*see* **FIG. 1**), a medium analysis part 14 which analyzes a liquid culture medium, a cell inspection removal part 15 which inspects the differentiated cells and performs removal of the differentiated cells having a bad state, a liquid storage supply part 16 which stores and supplies a liquid including a liquid culture medium or a proteolytic enzyme, an incubator part 17 which automatically adjusts one or all of a temperature, a humidity and a gas concentration within the housing 13, and a discharge part 18 for discharging downward from the housing 13 a waste liquid including a used liquid culture medium, a used cleaning liquid, a used reagent or the like used within the iPS cell establishing device 11. Furthermore, the iPS cell establishing device 11 includes a control part 19 connected to the medium analysis part 14, the cell inspection removal part 15, the liquid storage supply part 16, the incubator part 17 and the discharge part 18 so as to make communication therewith and configured to control them. The respective control parts 19, 29 and 39 are installed in an external device 90 such as, e.g., a personal computer or the like.

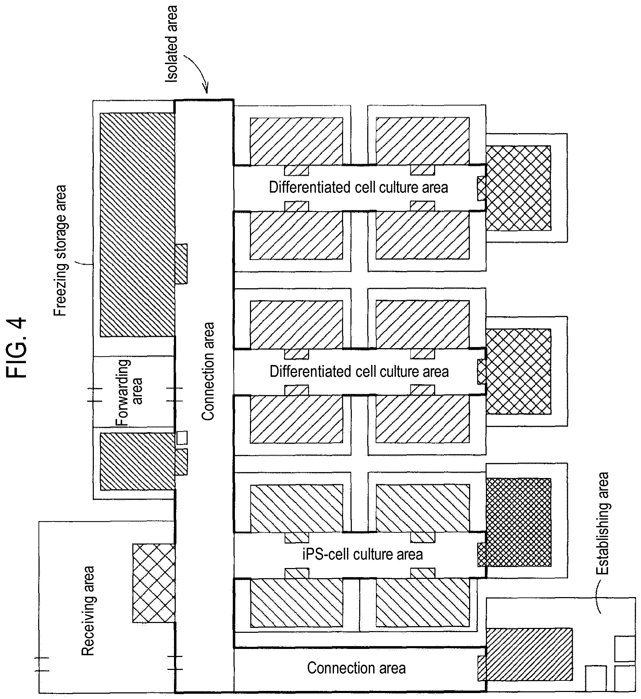

As illustrated in **FIG. 3A****,** the transfer part 60 of the present embodiment includes a holding portion 61 which holds the first airtight container 70 such that the first airtight container 70 is suspended downward. The transfer part 60 is configured to move along a rail 65 provided in a ceiling. Furthermore, the area in which the transfer part 60 moves is an isolated area covered with a wall surface and sterilized to some extent. In **FIGS. 1** and **4****,** the area indicated by thick lines is the isolated area. **FIG. 4** is a view corresponding to **FIG. 1** and is a view for explaining respective areas in the automatic culture system according to the present embodiment.

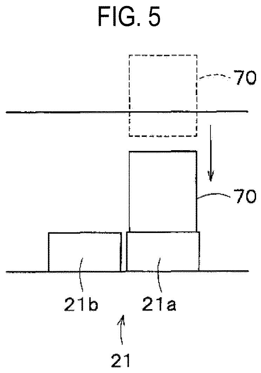

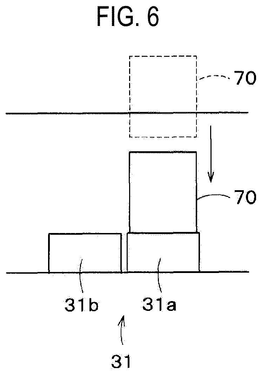

As illustrated in **FIG. 1****,** the iPS-cell automatic culture device 20 includes a loading part 21 provided within the isolated area and configured to load the first airtight container 70. As illustrated in **FIG. 5****,** the loading part 21 includes a cell loading/unloading part 21a for loading the iPS cells accommodated in the second airtight container 75 and for unloading the cultured iPS cells, and a material loading part 21b for loading materials. Similarly, as illustrated in **FIG. 1****,** the differentiated-cell automatic culture device 30 includes a loading part 31 provided within the isolated area and configured to load the first airtight container 70. As illustrated in **FIG. 6****,** the loading part 31 includes a cell loading/unloading part 31a for loading the iPS cells accommodated in the second airtight container 75 and for unloading the cultured differentiated cells, and a material loading part 31b for loading materials. In the present embodiment, the materials include a liquid culture medium, a reagent, a cleaning liquid, a culture plate, a vial, a filter, a needle, and so forth. Furthermore, as illustrated in **FIG. 1****,** the iPS cell establishing device 11 includes a loading part 12 provided within the isolated area and configured to load the first airtight container 70. By the way, each of the cell loading/unloading part 21a and the cell loading/unloading part 31a corresponds to a "cell loading part" of the claims. However, in the present embodiment, the cell loading/unloading part 21a serves to perform both the "loading" of the iPS cells and the "unloading" of the iPS cells. Furthermore, the cell loading/unloading part 31a serves to perform both the "loading" of the iPS cells or the differentiated cells and the "unloading" of the differentiated cells. Thus, the cell loading/unloading part 21a or the cell loading/unloading part 31a is a "loading/unloading part".

As illustrated in **FIG. 4****,** the isolated area includes an establishing area for establishing the iPS cells, an iPS cell culture area for culturing the iPS cells, a differentiated cell culture area for culturing the differentiated cells, and a connection area which interconnects the establishing area, the iPS cell culture area and the differentiated cell culture area. The loading part 21 of the iPS-cell automatic culture device 20 is provided within the iPS cell culture area. The loading part 31 of the differentiated-cell automatic culture device 30 is provided within the differentiated cell culture area. Furthermore, outside the isolated area, there are provided a receiving area where raw material cells such as somatic cells or the like serving as a source of the iPS cells are received and a forwarding area where the produced iPS cells and/or differentiated cells are forwarded.

As illustrated in **FIG. 1****,** the automatic culture system of the present embodiment may further include a temporary storage part 5 which temporarily stores the first airtight container 70. The temporary storage part 5 is configured so that one or all of the temperature, the humidity and the gas concentration within the temporary storage part 5 can be adjusted. For example, the temperature is adjusted to become about 37 degrees C. Furthermore, the gas concentration within the temporary storage part 5 is adjusted by appropriately adding CO₂ to the air. If necessary, the humidity may be adjusted so as to become about 100%.

Furthermore, one or all of the temperature, the humidity and the gas concentration within the first airtight container 70 may be made adjustable. Even in this case, for example, the temperature is adjusted to become about 37 degrees C. Moreover, the gas concentration within the first airtight container 70 is adjusted by appropriately adding CO₂ to the air. If necessary, the humidity may be adjusted so as to become about 100%. In the case where the second airtight container 75 is employed as in the present embodiment, it is possible to position the cells in a state in which the cells are sealed by the second airtight container 75. Thus, there is no need to particularly adjust the humidity within the first airtight container 70.

By the way, in the present embodiment, the materials composed of a liquid or a gas may be directly supplied to the iPS-cell automatic culture device 20 or the differentiated-cell automatic culture device 30 via a supply pipe not illustrated.

Furthermore, as illustrated in **FIG. 1****,** the automatic culture system of the present embodiment includes accommodation rooms 100 which correspond to the iPS-cell automatic culture devices 20 and the differentiated-cell automatic culture devices 30 and which accommodate the iPS-cell automatic culture devices 20 and the differentiated-cell automatic culture devices 30. That is to say, in the present embodiment, the iPS-cell automatic culture devices 20 and the differentiated-cell automatic culture devices 30 are accommodated in the corresponding accommodation rooms 100 in a one-to-one relationship. The isolated-area-side surfaces of the accommodation rooms 100 are opened. The housings 22 and 32 of the iPS-cell automatic culture devices 20 and the differentiated-cell automatic culture devices 30 are fitted to the openings. The loading part 21 of the iPS-cell automatic culture device 20 and the loading part 31 of the differentiated-cell automatic culture device 30 are positioned within the isolated area. A pathway is provided adjacent to each of the accommodation rooms 100. An entrance (not illustrated) through which a worker can enter and go out is provided at the point which does not face the isolated area.

As illustrated in **FIG. 1****,** the automatic culture system of the present embodiment includes a sterilizing device 1 for sterilizing the interior of the first airtight container 70, analysis devices 80 and 85 which receive the cells cultured in the automatic culture devices 20 and 30 from the loading parts 21 and 31 at predetermined timings and inspect the cells thus received, and a freezing storage device 40 which freezes and stores the iPS cells, the differentiated cells or both cultured in the automatic culture devices 20 and 30. There may be provided a plurality of freezing storage devices 40. A freezing storage area per se may be cooled to serve as a freezer. In the case where there is provided a plurality of freezing storage devices 40 or in the case where the freezing storage area per se serves as a freezer, a rail 65 may be provided in the ceiling of the freezing storage area so that the transfer part 60 can move along the rail 65. The aforementioned analysis devices 80 and 85 include an iPS-cell analysis device 80 which performs analysis of the iPS cells and a differentiated-cell analysis device 85 which performs analysis of the differentiated cells.

In the aspect illustrated in **FIG. 1****,** the loading part 41 of the freezing storage device 40 of the present embodiment and the loading parts 81 and 86 of the analysis devices 80 and 85 are provided in the isolated area. However, the entire freezing storage area may be provided within the isolated area in the case where there is provided a plurality of freezing storage devices 40 or in the case where the freezing storage area per se is cooled to serve as a freezer.

One example of the sterilizing device 1 may include sterilization which sterilizes the interior of the first airtight container 70 by supplying a high-temperature gas into the first airtight container 70. Another example of the sterilizing device 1 may include sterilization which sterilizes the interior of the first airtight container 70 by irradiating, for example, γ rays from the outside while keeping the first airtight container 70 in a sealed state. In addition, before the first airtight container 70 is loaded into the receiving area, the interior of the first airtight container 70 may be sterilized using, for example, γ rays or ultraviolet rays. There may be a case where the liquid culture medium or the like contains protein or the like which is broken by γ rays or ultraviolet rays. In this case, it is desirable that sterilization is performed by a sterilizing gas such as a hydrogen peroxide gas, a high-temperature gas or the like.

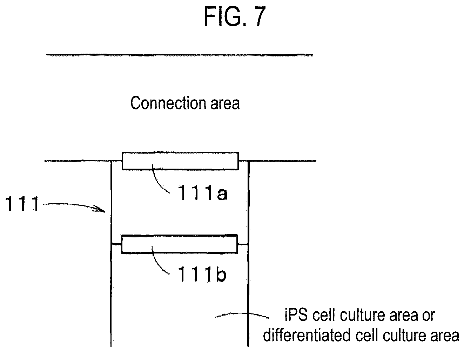

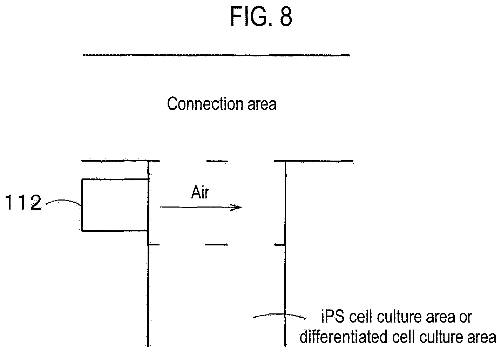

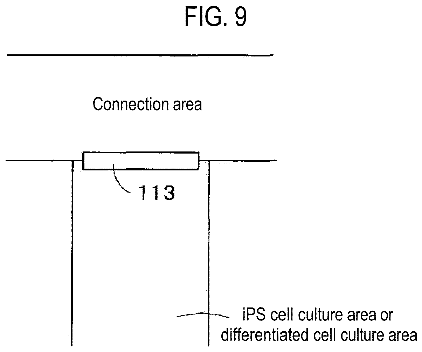

The automatic culture system of the present embodiment may include an isolation member which isolates a predetermined section in the isolation area. The isolation member is provided, for example, between the connection area and the iPS cell culture area and/or between the connection area and the differentiated cell culture area (*see* **FIG. 4**). One example of the isolation member is a double-entry door 111 (*see* **FIG. 7**). Another example of the isolation member is an air shower including an air blowing part 112 *(see* **FIG. 8**). A further example of the isolation member is a single-entry door 113 (*see* **FIG. 9**). By providing this isolation member, it is possible to prevent contamination and to increase the sterilization efficiency in an abnormal case.

In the case where the isolation member is the double-entry door 111 as illustrated in **FIG. 7****,** a configuration may be available, in which one door 111a is opened when the first airtight container 70 comes close from the side of one door 111a (at this time, the other door 111b is kept closed) and one door 111a is closed after the first airtight container 70 passes through one door 111a. In this configuration, the other door 111b is opened at the same time as the closure of one door 111a or after one door 111a is closed. Further, the first airtight container 70 passes through the other door 111b. Finally, the other door 111b is closed after the first airtight container 70 passes through the other door 111b. In the case where the first airtight container 70 comes close from the side of the other door 111b, the procedures opposite to those described above may be performed. By doing so, it is possible to reliably partition the respective areas within the isolated area. Even if contamination occurs within the isolated area by whatever reasons, it is possible to minimize a contaminated region.

In the case where the isolation member is the air shower as illustrated in **FIG. 8****,** it is not necessary to perform the door opening/closing operation performed in the double-entry door 111 described above. It is only necessary that an air having a positive pressure is always blown from the air blowing part 112. By doing so, it is possible to reliably partition the respective areas within the isolated area. Even if contamination occurs within the isolated area by whatever reasons, it is possible to prevent the widening of a contaminated region.

In the case where the isolation member is the single-entry door 113 as illustrated in **FIG. 9****,** the single-entry door 113 is normally opened. Only when necessary, the single-entry door 113 may be closed. In this aspect, the single-entry door 113 is automatically closed at the time point when contamination is detected within the isolated area. Thus, even if contamination occurs within the isolated area by whatever reasons, it is possible to prevent the widening of a contaminated region as far as possible.

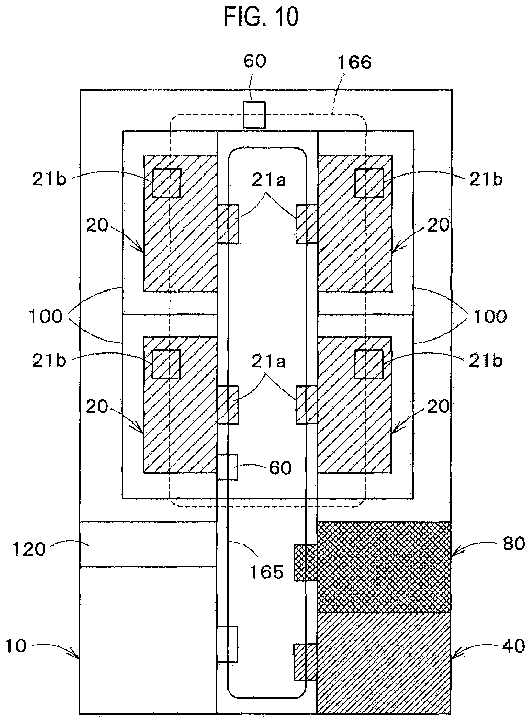

In the present embodiment, descriptions are made on a case in which both the iPS-cell automatic culture device 20 and the differentiated-cell automatic culture device 30 are provided. However, the automatic culture system may include only one of the iPS-cell automatic culture device 20 and the differentiated-cell automatic culture device 30 (This is directed to a second embodiment (*see* **FIG. 10**)).

### <<Method>>

An automatic culture method using the automatic culture system of the present embodiment will be described herein below.

### [Loading into Isolated Area]

Initially, descriptions will be made on one example of a process of loading cells and materials for culturing the cells into the isolated area.

First, in an area outside the isolated area, the second airtight container 75 which accommodates raw material cells such as somatic cells or the like is disposed within the first airtight container 70, the second airtight container 75 which accommodates materials such as a liquid culture medium, a chemical and the like is disposed within the first airtight container 70, or materials such as a filter, a needle and the like are directly disposed within the first airtight container 70.

Then, the interior of the first airtight container 70 is sterilized by a sterilizing gas supplied from the sterilizing device 1. Thereafter, the first airtight container 70 is closed into a sealed state. In the case where γ rays or ultraviolet rays are used for sterilization, the interior of the first airtight container 70 is sterilized after the first airtight container 70 is closed into a sealed state. Before the first airtight container 70 is loaded into the isolated area, one or all of the temperature, the humidity and the gas concentration within the first airtight container 70 may be adjusted.

Then, the first airtight container 70, the interior of which is sterilized, is mounted in an input part 2 leading to the isolated area (*see* **FIG. 1**). The input part 2 is formed of, for example, a double-entry door. If the first airtight container 70 is mounted, one door positioned at the side of the receiving area is opened (at this time, the other door positioned at the side of the isolated area is kept closed). The first airtight container 70 is introduced into between the other door and one door. The other door is opened after one door is closed. Thereafter, the first airtight container 70 is received by the transfer part 60 and is transferred within the isolated area. After the first airtight container 70 is loaded into the isolated area, the other door is closed. A sterilizing process may be performed while both doors are kept closed.

In the case where the second airtight container 75 which accommodates raw material cells is accommodated in the first airtight container 70, the first airtight container 70 is transferred to the raw material storage device 10 by the transfer part 60. Then, in the raw material storage device 10, the second airtight container 75 is taken out from the interior of the first airtight container 70. Alternatively, the raw material cells may be manually transported into the accommodation room 100 of the establishing area via an entrance not illustrated. A worker may perform a work with the raw material storage device 10 disposed within the accommodation room 100, thereby establishing iPS cells. Furthermore, the receiving area may serve as the establishing area. In this case, it is possible to reduce the areas where a worker performs a work. Thus, the management becomes easy.

On the other hand, in the case where the second airtight container 75 which accommodates materials or the materials per se is accommodated in the first airtight container 70, the first airtight container 70 is transferred to the loading part 21 of a pre-designated iPS-cell automatic culture device 20 (more specifically, the material loading part 21b) or the loading part 31 of the differentiated-cell automatic culture device 30 (more specifically, the material loading part 31b) by the transfer part 60. Then, in the iPS-cell automatic culture device 20 or the differentiated-cell automatic culture device 30, the second airtight container 75 which accommodates materials or the materials per se is taken out from the interior of the first airtight container 70 and is loaded into the iPS-cell automatic culture device 20 or the differentiated-cell automatic culture device 30.

### [From Establishing to Forwarding]

Subsequently, descriptions will be made on a process during which the iPS cells established in the raw material storage device 10 are differentiated and forwarded.

One or more second airtight containers 75 which accommodate the iPS cells established in the raw material storage device 10 are disposed within the first airtight container 70 and are sealed. At this time, one or all of the temperature, the humidity and the gas concentration within the first airtight container 70 may be adjusted.

The first airtight container 70 which accommodates the second airtight containers 75 is transferred to the loading part 21 of the iPS-cell automatic culture device 20 (more specifically, the cell loading/unloading part 21a). Then, in the iPS-cell automatic culture device 20, the second airtight containers 75 are taken out from the first airtight container 70.

The iPS cells loaded into the iPS-cell automatic culture device 20 in this way are cultured within the iPS-cell automatic culture device 20. During the culture, the replacement of a liquid culture medium is appropriately and automatically performed, and the subculture of the iPS cells is appropriately and automatically performed. Furthermore, one or all of the temperature, the humidity and the gas concentration of the incubator part 27 may be adjusted. In the meantime, the iPS-cell automatic culture devices 20 are divided for each cell donor. In the case where iPS cells of a person A are cultured in a certain iPS-cell automatic culture device 20, iPS cells of a person other than A or iPS cells of an animal are not cultured in that iPS-cell automatic culture device 20.

Some of the iPS cells cultured in the iPS-cell automatic culture device 20 are appropriately taken out from the iPS-cell automatic culture device 20 by the transfer part 60 and are transferred to the loading part 81 of the iPS-cell analysis device 80. Then, the culture state (e.g., the DNA state) of the iPS cells is analyzed within the iPS-cell analysis device 80. Unlike the inspection performed within the iPS-cell automatic culture device 20, the analysis performed in the iPS-cell analysis device 80 is a destructive inspection. Thus, the iPS cells used in the analysis are discarded without being returned to the iPS-cell automatic culture device 20.

If the iPS cells are cultured in the iPS-cell automatic culture device 20, one or more second airtight containers 75 which accommodate the iPS cells are disposed within the first airtight container 70 and are sealed. At this time, one or all of the temperature, the humidity and the gas concentration within the first airtight container 70 may be adjusted by the iPS-cell automatic culture device 20.

The first airtight container 70 which accommodates the second airtight containers 75 is transferred to the loading part 31 of the differentiated-cell automatic culture device 30 (more specifically, the cell loading/unloading part 31a) by the transfer part 60. In the differentiated-cell automatic culture device 30, the second airtight containers 75 are taken out from the interior of the first airtight container 70.

The iPS cells loaded into the differentiated-cell automatic culture device 30 in this way are differentiated and cultured within the differentiated-cell automatic culture device 30. During the culture, the replacement of a liquid culture medium is appropriately and automatically performed, and the subculture of the differentiated cells is appropriately and automatically performed. Furthermore, a liquid culture medium including a differentiation inducing factor may be appropriately supplied. Moreover, one or all of the temperature, the humidity and the gas concentration of the incubator part 37 may be adjusted. In the meantime, the differentiated-cell automatic culture devices 30 are divided for each cell donor. In the case where A's differentiated cells are cultured in a certain differentiated-cell automatic culture device 30, differentiated cells of a person other than A or differentiated cells of an animal are not cultured in that differentiated-cell automatic culture device 30.

Some of the differentiated cells cultured in the differentiated-cell automatic culture device 30 are appropriately taken out from the differentiated-cell automatic culture device 30 by the transfer part 60 and are transferred to the loading part 86 of the differentiated-cell analysis device 85. Then, the culture state (e.g., the DNA state) or the like of the differentiated cells is analyzed within the differentiated-cell analysis device 85. Unlike the inspection performed within the differentiated-cell automatic culture device 30, the analysis performed in the differentiated-cell analysis device 85 is a destructive inspection. Thus, the differentiated cells used in the analysis are discarded without being returned to the differentiated-cell automatic culture device 30.

If the differentiated cells are cultured in the differentiated-cell automatic culture device 30, one or more second airtight containers 75 which accommodate the differentiated cells are disposed within the first airtight container 70 and are sealed. At this time, one or all of the temperature, the humidity and the gas concentration within the first airtight container 70 may be adjusted by the differentiated-cell automatic culture device 30.

In the case of freezing and storing the differentiated cells, the first airtight container 70 which accommodates the second airtight containers 75 which accommodate the differentiated cells is transferred to the freezing storage device 40 by the transfer part 60. Then, in the freezing storage device 40, the second airtight containers 75 are taken out from the interior of the first airtight container 70. The differentiated cells are stored within the freezing storage device 40 in a state in which the differentiated cells are accommodated in the second airtight containers 75. Instead of taking out the second airtight containers 75 from the interior of the first airtight container 70 in the aforementioned manner, the second airtight containers 75 and the differentiated cells may be kept accommodated in the first airtight container 70 and may be stored within the freezing storage device 40 in that state.

In the case where the cultured iPS cells are frozen and stored without differentiating them, the first airtight container 70 which accommodates the second airtight containers 75 which accommodate the iPS cells is transferred to the freezing storage device 40 by the transfer part 60. Then, in the freezing storage device 40, the second airtight containers 75 are taken out from the interior of the first airtight container 70. The iPS cells are stored within the freezing storage device 40 in a state in which the iPS cells are accommodated in the second airtight containers 75. Even in this case, instead of taking out the second airtight containers 75 from the interior of the first airtight container 70, the second airtight containers 75 and the iPS cells may be kept accommodated in the first airtight container 70 and may be stored within the freezing storage device 40 in that state.

When forwarding the iPS cells or the differentiated cells stored in the freezing storage device 40, the second airtight containers 75 which accommodate the iPS cells or the differentiated cells are accommodated in the first airtight container 70. The first airtight container 70 is transferred from the freezing storage device 40 to the forwarding area by the transfer part 60. In the case where the first airtight container 70 per se is stored within the freezing storage device 40, the first airtight container 70 is taken out from the freezing storage device 40 as it is. The first airtight container 70 is transferred from the freezing storage device 40 to the forwarding area by the transfer part 60. If the first airtight container 70 is transferred to the forwarding area in this way, the iPS cells or the differentiated cells are forwarded using the first airtight container 70 per se or using the second airtight containers 75 taken out from the first airtight container 70.

In the case where the forwarding destination of the iPS cells or the differentiated cells (e.g., a hospital or a factory) is located nearby or positioned in the neighborhood, it is not necessary to freeze the iPS cells or the differentiated cells. For that reason, in this case, instead of freezing and storing the iPS cells or the differentiated cells, the second airtight containers 75 which accommodate the iPS cells taken out from the iPS-cell automatic culture device 20 are accommodated in the first airtight container 70 and are transferred to the forwarding area by the transfer part 60, or the second airtight containers 75 which accommodate the differentiated cells taken out from the differentiated-cell automatic culture device 30 are accommodated in the first airtight container 70 and are transferred to the forwarding area by the transfer part 60. Thereafter, the iPS cells or the differentiated cells are forwarded from the forwarding area to the adjacent or close forwarding destination using the first airtight container 70 per se or using the second airtight containers 75 taken out from the first airtight container 70.

### <<Effects>>

Next, the effects achieved by the present embodiment configured as above, which are not yet described but are especially important, will be described.

According to the present embodiment, the automatic culture devices 20 and 30 automatically adjust at least one of the temperature, the humidity and the gas concentration within the automatic culture devices 20 and 30 and automatically perform the replacement and subculture of the culture medium. Furthermore, the iPS cells are transferred by the transfer part 60 between the raw material storage device 10 and the iPS-cell automatic culture device 20 and between the iPS-cell automatic culture device 20 and the differentiated-cell automatic culture device 30. It is therefore possible to completely automatically perform the process of culturing and differentiating the established iPS cells.

More specifically, in the present embodiment, the iPS-cell automatic culture device 20 automatically adjusts at least one of the temperature, the humidity and the gas concentration within the iPS-cell automatic culture device 20 and automatically performs the replacement and subculture of the culture medium. Thus, once the iPS cells are loaded into the iPS-cell automatic culture device 20, it is possible to perform the culture of the iPS cells in the iPS-cell automatic culture device 20 without human intervention.

Furthermore, in the present embodiment, the differentiated-cell automatic culture device 30 automatically adjusts at least one of the temperature, the humidity and the gas concentration within the differentiated-cell automatic culture device 30 and automatically performs the replacement and subculture of the culture medium. Thus, once the iPS cells are loaded into the differentiated-cell automatic culture device 30, it is possible to perform the culture of the differentiated cells in the differentiated-cell automatic culture device 30 without intervention of the hand of a man.

Furthermore, in the present embodiment, when the cells are not positioned within the devices such as the raw material storage device 10 including the iPS cell establishing device 11, the iPS-cell automatic culture device 20, the differentiated-cell automatic culture device 30, the freezing storage device 40 and the like, the cells are necessarily kept in a sealed state in the first airtight container 70. Thus, there is no need to maintain high cleanliness in the out-of-device areas such as the isolated area and the like. As a result, it is possible to suppress an increase in the equipment cost, the maintenance cost or the like.

Furthermore, in the present embodiment, the iPS cell establishing device 11 and the automatic culture devices 20 and 30 include the discharge parts 18, 28 and 38 for discharging the waste liquid, which are separately formed from the loading parts 12, 21 and 31, so that the route for loading the cells or the materials and the route for discharging the waste liquid do not intersect each other. This makes it possible to reduce the risk of the cells or the materials being contaminated by the waste liquid. Moreover, the waste liquid discharged by the discharge parts 18, 28 and 38 is discharged downward from the housings 13, 22 and 32 so that the backflow of the waste liquid can be prevented. When discharging the waste liquid downward, it may be possible to discharge the waste liquid to the downstairs. Waste materials may be discharged from the material loading parts 21b and 31b. Alternatively, similar to the waste liquid, the waste materials may be discharged downward from the discharge parts 28 and 38. Even in this case, the waste materials may be discharged to the downstairs. Examples of the waste materials may include a used second airtight container 75, a filter used in the analysis devices 80 and 85a and the like. When discharging the waste liquid by the discharge parts 18, 28 and 38, the waste liquid may be discharged at all times or may be discharged by pressure-feeding the waste liquid after a certain amount of the waste liquid is collected.

In the present embodiment, there are provided the second airtight containers 75 which accommodate the cells. The second airtight containers 75 are accommodated in the first airtight container 70 and are transferred in this state. Thus, the cells are kept in a sealed state by the first airtight container 70 and are also kept in a sealed state by the second airtight containers 75. This makes it possible to reliably keep the cells in a sealed state. Inside the devices such as the raw material storage device 10 including the iPS cell establishing device 11, the iPS-cell automatic culture device 20, the differentiated-cell automatic culture device 30, the freezing storage device 40 and the like, the cells are sealed within the second airtight containers 75. It is therefore possible to prevent the cells from being contaminated by whatever reasons within the devices.

In the case where the automatic culture system of the present embodiment employs an aspect in which the sterilizing device 1 for sterilizing the interior of the first airtight container 70 is provided, it is possible to sterilize the interior of the first airtight container 70 by the sterilizing device 1 within the automatic culture system.

In the present embodiment, in the case where there is employed an aspect capable of adjusting one or all of the temperature, the humidity and the gas concentration within the first airtight container 70, an environment preferable for the cells or the materials can be maintained even when the cells are moved within the isolated area.

Furthermore, in the case where the automatic culture system of the present embodiment employs an aspect in which the temporary storage part 5 for temporarily storing the first airtight container 70 is provided, it is possible to temporarily store the first airtight container 70 in the temporary storage part 5 even when the state in which the cells (the raw material cells, the iPS cells, the differentiated cells, etc.) or the materials need to be accommodated within the first airtight container 70 is sustained for a long period of time. Thus, it is possible to maintain an environment preferable for the cells or the materials. In the temporary storage part 5, the cells or the materials may not be taken out from the first airtight container 70 and may be stored in a state in which the cells or the materials are accommodated in the first airtight container 70. However, the present disclosure is not limited to this aspect. The cells or the materials may be temporarily stored in the temporary storage part 5 in a state in which the cells or the materials are taken out from the first airtight container 70 (for example, a state in which the cells or the materials are accommodated in the second airtight containers 75).

Furthermore, in the case where the loading parts 21 and 31 of the automatic culture devices 20 and 30 of the present embodiment include the cell loading/unloading parts 21a and 31a for loading the first airtight container 70 which accommodates the cells and for unloading the cultured cells and the material loading parts 21b and 31b for loading the materials for the culture of the cells, the route for loading the cells or the materials into the automatic culture devices 20 and 30 may be divided into a cell route and a material route. This makes it possible to prevent occurrence of a situation in which the materials adhere to the produced cells (the iPS cells, the differentiated cells, etc.) due to whatever causes.

In the present embodiment, the automatic culture devices 20 and 30 are accommodated in the corresponding accommodation rooms 100 in a one-to-one relationship. The isolated-area-side surfaces of the accommodation rooms 100 are opened. The housings 22 and 32 of the automatic culture devices 20 and 30 are fitted to the openings. The loading parts 21 and 31 of the automatic culture devices 20 and 30 are positioned within the isolated area. On the other hand, the entrance through which a worker can enter and go out is provided at the point of each of the accommodation rooms 100 which does not face the isolated area. Thus, even if a worker enters through the entrance, for example, when the use of the automatic culture devices 20 and 30 is completed and the automatic culture devices 20 and 30 need to be sterilized, the isolated area and the worker-entered accommodation room 100 are still isolated from each other. This is beneficial in that there is no need to re-sterilize the isolated area. Since the automatic culture devices 20 and 30 are accommodated in the corresponding accommodation rooms 100 in a one-to-one relationship, even if a worker enters a certain accommodation room 100 when sterilizing the automatic culture devices 20 and 30 accommodated in that accommodation room 100, other accommodation rooms 100 are not affected by the entry and exit of the worker. This is beneficial in that there is no need to re-sterilize other accommodation rooms 100.

### Second Embodiment

Next, descriptions will be made on a second embodiment of the present disclosure.

In the second embodiment, a route for transferring cells and a route for transferring materials are different from each other and are not mixed with each other.

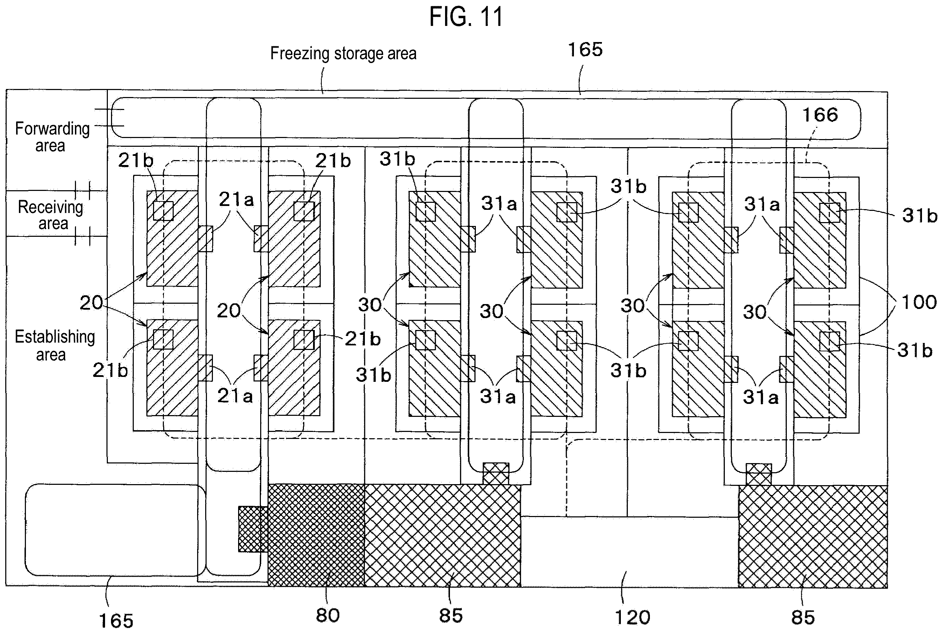

Other configurations of the second embodiment are substantially identical with those of the first embodiment. In the second embodiment, parts identical with those of the first embodiment are designated by like reference symbols with the detailed descriptions thereof omitted. **FIG. 10** is a schematic top plan view illustrating the configuration of an automatic culture system which includes a culture area where iPS cells are cultured and which does not include a differentiation area where iPS cells are differentiated. **FIG. 11** is a schematic top plan view illustrating the configuration of an automatic culture system which includes both a culture area where iPS cells are cultured and a differentiation area where iPS cells are differentiated.

In the present embodiment, the route indicated by a "solid line" in **FIGS. 10** and **11** is a cell transfer route 165 (more specifically, a cell transfer rail) for transferring cells. The route indicated by a "dot line" in **FIGS. 10** and **11** is a material transfer route 166 (more specifically, a material transfer rail) for unloading materials from a material stock part 120 and for transferring the materials. In the aspect illustrated in **FIG. 11****,** the area extending in the left-right direction at the uppermost side in **FIG. 11** (which is not illustrated in **FIG. 11**) is a freezing storage area where a freezing storage device is installed. In the freezing storage area, iPS cells or differentiated cells are frozen and stored.

In the present embodiment, a transfer part 60 which transfers cells is moved along the cell transfer route 165 provided within a cell transfer area. A transfer part 60 which transfers materials is moved along the material transfer route 166 provided within a material transfer area. In **FIGS. 10** and **11****,** at the points where the solid line and the dot line meet, the cell transfer route 165 and the material transfer route 166 three-dimensionally intersect each other and are separated from each other. That is to say, for example, the material transfer route 166 is covered with a cylindrical wall surface. Inside the wall surface, a first airtight container 70 which accommodates materials is transferred along the material transfer route 166. Outside the wall surface, a first airtight container 70 which accommodates cells is transferred along the cell transfer route 165.

Furthermore, in the present embodiment, the cell loading/unloading part 21a of the iPS-cell automatic culture device 20 which holds and loads the first airtight container 70 that accommodates cells (more specifically, accommodates the second airtight container 75 which accommodates cells) and the material loading part 21b of the iPS-cell automatic culture device 20 which loads the first airtight container 70 that accommodates materials (more specifically, accommodates the second airtight container 75 which accommodates materials, or accommodates the materials per se) are positioned in completely different positions. More specifically, the cell loading/unloading part 21a is positioned within the cell transfer area. The first airtight container 70 transferred along the cell transfer route 165 by the transfer part 60 is positioned in a lower position. Thus, the first airtight container 70 is mounted on the cell loading/unloading part 21a. On the other hand, the material loading part 21b is positioned within the material transfer area. The first airtight container 70 transferred along the material transfer route 166 by the transfer part 60 is positioned in a lower position. Thus, the first airtight container 70 is mounted on the material loading part 21b.

Even in the present embodiment, the materials composed of a liquid or a gas may be directly supplied to the iPS-cell automatic culture device 20 and the differentiated-cell automatic culture device 30 via a supply pipe. Furthermore, a refrigeration part may be disposed near the iPS-cell automatic culture device 20 and the differentiated-cell automatic culture device 30. A liquid culture medium cooled to a temperature of about 4 degrees C by the refrigeration part may be supplied to the iPS-cell automatic culture device 20 and the differentiated-cell automatic culture device 30 via a supply pipe. Furthermore, waste materials may be unloaded from the material loading parts 21b and 31b rather than the discharge parts 28 and 38. In this case, each of the material loading parts 21b and 31b serves as a material loading/unloading part.

According to the second embodiment described above, it is possible to reliably separate the cell transfer area where cells may exist and the material transfer area where materials other than cells may exist. In this way, if the space where cells and materials are mixed with each other is limited to only the interior of the automatic culture devices 20 and 30, it is possible to reliably prevent the produced cells (the iPS cells, the differentiated cells, etc.) from being contaminated by the materials due to whatever causes.

Finally, the foregoing descriptions of the respective embodiments and the disclosure of the drawings are nothing more than one example for describing the present disclosure recited in the claims.

## Claims

1. An automatic culture system, comprising:
a transfer part (60) configured to transfer a first airtight container (70) which accommodates cells in a sealed state; and
an automatic culture device (20, 30) having a loading part (21, 31) provided within an isolated area and configured to receive the first airtight container (70) transferred by the transfer part (60), take out the cells from the first airtight container (70) and culture the taken-out cells,
wherein the transfer part (60) includes a ceiling, a rail (65) provided in the ceiling and a holding portion (61) configured to hold the first airtight container (70) such that the first airtight container (70) is suspended downward from the rail (65),
wherein the isolated area is partitioned into a plurality of areas by an isolation member,
wherein the plurality of areas include at least two areas being partitioned by the isolation member, and
wherein the ceiling includes ceilings of the at least two areas, and the rail (65) extends between the ceilings of the at least two areas, and the transfer part (60) is configured to move along the rail (65) and pass through the isolation member while holding the first airtight container (70), wherein the automatic culture device (20, 30) is configured to automatically perform replacement of a culture medium.

2. The system of Claim 1, wherein the isolation member is one of a single-entry door, a double-entry door and an air shower.

3. The system of any one of Claims 1 or 2, wherein the first airtight container (70) is configured to accommodate materials including a liquid culture medium, a reagent, a cleaning liquid, a culture plate, a vial, a filter and a needle,
wherein the transfer part (60) includes a cell transfer route (165) through which the first airtight container (70) accommodating the cells is transferred and a material transfer route (166) through which the first airtight container (70) accommodating the materials is transferred, the cell transfer route (165) and the material transfer route (166) being different from each other.

4. The system of any one of Claims 1 to 3, wherein the cell transfer route (165) and the material transfer route (166) three-dimensionally intersect each other.

5. The system of any one of Claims 1 to 4, wherein the automatic culture device (20, 30) is configured to automatically perform subculture of the cells.

6. The system of any one of Claims 1 to 5, wherein the first airtight container (70) is transferred by the transfer part (60) in a state in which a second airtight container (75) that accommodates the cells is accommodated within the first airtight container (70).

7. The system of Claim 6, wherein the first airtight container (70) is transferred by the transfer part (60) in a state in which a plurality of second airtight containers (75) is accommodated within the first airtight container (70).

8. The system of Claim 6 or 7, wherein the automatic culture device (20, 30) is configured to receive the first airtight container (70) which accommodates the second airtight container (75) and which is transferred by the transfer part (60), take out the second airtight container (75) from the first airtight container (70), and automatically culture the cells in a state in which the cells are accommodated in the taken-out second airtight container (75).

9. The system of any one of Claims 1 to 8, further comprising:
a sterilizing device (1) configured to sterilize an interior of the first airtight container (70) which accommodates the cells.

10. The system of Claim 9, wherein the sterilizing device (1) is configured to sterilize the interior of the first airtight container (70) which accommodates the cells, by supplying a sterilizing gas into the first airtight container (70).

11. The system of any one of Claims 1 to 10, wherein the automatic culture device (20, 30) includes a discharge part (18, 28, 38) configured to discharge at least a waste liquid.

12. The system of any one of Claims 1 to 11, wherein at least one of a temperature, a humidity and a gas concentration within the first airtight container (70) is adjustable.

13. The system of any one of Claims 1 to 12, further comprising:
a temporary storage part (5) configured to temporarily store the first airtight container (70),
wherein at least one of a temperature, a humidity and a gas concentration within the temporary storage part (5) is adjustable.

14. The system of any one of Claims 1 to 13, wherein the loading part (21,31) includes a cell loading part (21a, 31a) configured to load the first airtight container (70) which accommodates the cells and a material loading part (21b, 31b) configured to load materials.

15. The system of any one of Claims 1 to 14, wherein the automatic culture device (20, 30) includes a plurality of automatic culture devices,
the system further comprises a plurality of accommodation rooms (100) configured to accommodate the automatic culture devices (20, 30), and
the automatic culture devices (20, 30) are respectively accommodated in the corresponding accommodation rooms (100) in a one-to-one relationship.

16. An automatic culture method, comprising:
transferring, by a transfer part (60), a first airtight container (70) which accommodates cells in a sealed state;
receiving, by an automatic culture device (20, 30) having a loading part (21, 31) provided within an isolated area, the first airtight container (70) transferred by the transfer part (60) and taking out the cells from the first airtight container (70); and
culturing, by the automatic culture device (20, 30), the taken-out cells,
wherein the transfer part (60) includes a ceiling, a rail (65) provided in the ceiling and a holding portion (61) configured to hold the first airtight container (70) such that the first airtight container (70) is suspended downward from the rail (65),
wherein the isolated area is partitioned into a plurality of areas by an isolation member,
wherein the plurality of areas include at least two areas being partitioned by the isolation member, and
wherein the ceiling includes ceilings of the at least two areas, and the rail (65) extends between the ceilings of the at least two areas, and the transfer part (60) is configured to move along the rail (65) and pass through the isolation member while holding the first airtight container (70),
wherein the automatic culture device is configured to automatically adjust at least one of a temperature, a humidity and a gas concentration within the automatic culture device and to automatically perform replacement of a culture medium.

## Patentansprüche

1. Automatisches Kultursystem, mit:
einem Transfer-Teil (60), der dazu ausgebildet ist, einen ersten luftdichten Container (70), welcher in verschlossenem Zustand Zellen beherbergt, zu transferieren; und
eine automatische Kulturvorrichtung (20, 30) mit einem in einem isolierten Bereich bereitgestellten Beschickungs-Teil (21, 31), das dazu ausgebildet ist, den ersten luftdichten Container (70), der vom Transfer-Teil (60) transferiert wird, zu empfangen, die Zellen aus dem ersten luftdichten Container (70) zu entnehmen, und die entnommenen Zellen zu entnehmen,
wobei der Transfer-Teil (60) eine Decke, eine in der Decke bereitgestellte Schiene (65) und einen Halterungsbereich (61) aufweist, der dazu ausgebildet ist, den ersten luftdichten Container (70) derart zu haltern, dass der erste luftdichte Container (70) von der Schiene (65) nach unten hängt,
wobei der isolierte Bereich durch ein Isolierglied in mehrere Bereiche unterteilt ist,
wobei die mehreren Bereiche wenigstens zwei Bereiche beinhalten, die durch das Isolierglied unterteilt werden, und
wobei die Decke die Decken der wenigstens zwei Bereiche beinhaltet, und die Schiene (65) sich zwischen den Decken der wenigstens zwei Bereiche erstreckt, und der Transfer-Teil (60) dazu ausgebildet ist, sich entlang der Schiene (65) zu bewegen und durch das Isolierglied hindurchzutreten, während er den ersten luftdichten Container (70) haltert, wobei die automatische Kulturvorrichtung (20, 30) dazu ausgebildet ist, automatisch einen Austausch eines Kulturmediums vorzunehmen.

2. System nach Anspruch 1, wobei das Isolierglied eine Single-Entry-Tür, eine Double-Entry-Tür oder eine Luftdusche ist.

3. System nach Anspruch 1 oder 2,
wobei der erste luftdichte Container (70) dazu ausgebildet ist, Materialien einschließlich eines flüssigen Kulturmediums, eines Reagens, einer Reinigungsflüssigkeit, einer Kulturplatte, einer Phiole, eines Filters und einer Nadel zu beherbergen,
wobei der Transfer-Teil (60) eine Zelltransferroute (165) beinhaltet, über welche der die Zellen beherbergende erste luftdichte Container (70) transferiert wird, und eine Materialtransferroute (166), über welche der die Materialien beherbergende erste luftdichte Container (70) transferiert wird, wobei die Zelltransferroute (165) und die Materialtransferroute (166) voneinander verschieden sind.

4. System nach einem der Ansprüche 1 bis 3, wobei die Zelltransferroute (165) und die Materialtransferroute (166) einander drei-dimensional schneiden.

5. System nach einem der Ansprüche 1 bis 4, wobei die automatische Kulturvorrichtung (20, 30) dazu ausgebildet ist, automatisch Subkulturen der Zellen durchzuführen.

6. System nach einem der Ansprüche 1 bis 5, wobei der erste luftdichte Container (70) mit dem Transfer-Teil (60) in einem Zustand transferiert wird, in dem ein zweiter luftdichter Container (75), der die Zellen beherbergt, innerhalb des ersten luftdichten Containers (70) beherbergt ist.

7. System nach Anspruch 6, wobei der erste luftdichte Container (70) mit dem Transfer-Teil (60) in einem Zustand transferiert wird, in dem mehrere zweite luftdichte Container (75) innerhalb des ersten luftdichten Containers (70) beherbergt sind.

8. System nach Anspruch 6 oder 7, wobei die automatische Kulturvorrichtung (20, 30) dazu ausgebildet ist, den ersten luftdichten Container (70) zu empfangen, der den zweiten luftdichten Container (75) beherbergt und der von dem Transfer-Teil (60) transferiert wird, den zweiten luftdichten Container (75) aus dem ersten luftdichten Container (70) zu entnehmen, und automatisch die Zellen in einem Zustand zu kultivieren, in welchem die Zellen in dem entnommenen zweiten luftdichten Container (75) beherbergt sind.

9. System nach einem der Ansprüche 1 bis 8, ferner aufweisend:
eine Sterilisiervorrichtung (1), die dazu ausgebildet ist, das Innere des ersten luftdichten Containers (70), welcher die Zellen beherbergt, zu sterilisieren.

10. System nach Anspruch 9, wobei die Sterilisiervorrichtung (1) dazu ausgebildet ist, das Innere des ersten luftdichten Containers (70), welcher die Zellen beherbergt, durch Einführen eines sterilisierenden Gases in den ersten luftdichten Container (70) zu sterilisieren.

11. System nach einem der Ansprüche 1 bis 10, wobei die automatische Kulturvorrichtung (20, 30) einen Ausgabe-Teil (18, 28, 38) aufweist, der dazu ausgebildet ist, zumindest einen Flüssigabfall abzulassen.

12. System nach einem der Ansprüche 1 bis 11, wobei die Temperatur, die Humidität und/oder eine Gaskonzentration innerhalb des ersten luftdichten Containers (70) einstellbar sind.

13. System nach einem der Ansprüche 1 bis 12, ferner aufweisend:
einen Zwischenlager-Teil (5), der dazu ausgebildet ist, temporär den ersten luftdichten Container (70) zu lagern,
wobei die Temperatur, die Humidität und/oder eine Gaskonzentration innerhalb des Zwischenlager-Teils (5) einstellbar sind.

14. System nach einem der Ansprüche 1 bis 13, wobei der Beschickungs-Teil (21, 31) einen Zellbeschickungs-Teil (21a, 31a) beinhaltet, der dazu ausgebildet ist, den ersten luftdichten Container (70) zu beschicken, der die Zellen beherbergt, und einen Materialbeschickungs-Teil (21b, 31b) beinhaltet, der dazu ausgebildet ist, Materialien zu beschicken.

15. System nach einem der Ansprüche 1 bis 14, wobei die automatische Kulturvorrichtung (20, 30) mehrere automatische Kulturvorrichtungen beinhaltet,
wobei das System ferner mehrere Beherbergungsräume (100) aufweist, die dazu ausgebildet sind, die automatischen Kulturvorrichtungen (20, 30) zu beherbergen, und
wobei die automatischen Kulturvorrichtungen (20, 30) jeweils in einer Eins-zu-Eins-Beziehung in den entsprechenden Beherbergungsräumen (100) beherbergt sind.

16. Automatisches Kulturverfahren, beinhaltend:
transferieren, durch den Transfer-Teil (60), eines ersten luftdichten Containers (70), welcher in einem verschlossenen Zustand Zellen beherbergt;
Empfangen, durch eine automatische Kulturvorrichtung (20, 30) mit einem in einem isolierten Bereich bereitgestellten Beschickungs-Teil (21, 31), des durch den Transfer-Teil (60) transferierten ersten luftdichten Containers (70), und Entnehmen der Zellen aus dem ersten luftdichten Container (70); und
Kultivieren, durch die automatische Kulturvorrichtung (20,30), der entnommene Zellen,
wobei der Transfer-Teil (60) eine Decke, eine an der Decke bereitgestellte Schiene (65) und einen Halterungsbereich (61) aufweist, der dazu ausgebildet ist, den ersten luftdichten Container (70) derart zu haltern, dass der erste luftdichte Container (70) von der Schiene (65) nach unten hängt,
wobei der isolierte Bereich durch ein Isolierglied in mehrere Bereiche unterteilt ist,
wobei die mehreren Bereiche wenigstens zwei durch das Isolierglied voneinander getrennte Bereiche beinhalten, und
wobei die Decke die Decken der wenigstens zwei Bereiche beinhaltet, und die Schiene (65) sich zwischen den Decken der wenigstens zwei Bereiche erstreckt, und der Transfer-Teil (60) dazu ausgebildet ist, sich entlang der Schiene (65) zu bewegen und durch das Isolierglied hindurchzutreten, während er den ersten luftdichten Container (70) haltert.

## Revendications

1. Système de culture automatique, comprenant :
une partie de transfert (60) configurée pour transférer un premier récipient étanche à l'air (70) qui reçoit des cellules dans un état scellé ; et
un dispositif de culture automatique (20, 30) ayant une partie de chargement (21, 31) agencée à l'intérieur d'une zone isolée et configuré pour recevoir le premier récipient étanche à l'air (70) transféré par la partie de transfert (60), extraire les cellules du premier récipient étanche à l'air (70) et mettre en culture les cellules extraites,
dans lequel la partie de transfert (60) inclut un plafond, un rail (65) agencé dans le plafond et une portion de maintien (61) configurée pour maintenir le premier récipient étanche à l'air (70) de telle sorte que le premier récipient étanche à l'air (70) soit suspendu vers le bas à partir du rail (65), dans lequel la zone isolée est segmentée en une pluralité de zones par un organe d'isolement,
dans lequel la pluralité de zones inclut au moins deux zones segmentées par l'organe d'isolement, et
dans lequel le plafond inclut des plafonds des au moins deux zones, et le rail (65) s'étend entre les plafonds des au moins deux zones, et la partie de transfert (60) est configurée pour se déplacer le long du rail (65) et passer à travers l'organe d'isolement tout en maintenant le premier récipient étanche à l'air (70), dans lequel le dispositif de culture automatique (20, 30) est configuré pour effectuer automatiquement un remplacement d'un milieu de culture.

2. Système selon la revendication 1, dans lequel l'organe d'isolement est l'un parmi une porte à entrée simple, une porte à entrée double et une douche à air.

3. Système selon l'une quelconque des revendications 1 ou 2,
dans lequel le premier récipient étanche à l'air (70) est configuré pour recevoir des matières incluant un milieu de culture liquide, un réactif, un liquide de nettoyage, une plaque à culture, un flacon, un filtre et une aiguille,
dans lequel la partie de transfert (60) inclut un trajet de transfert de cellules (65) par l'intermédiaire duquel le premier récipient étanche à l'air (70) recevant les cellules est transféré et un trajet de transfert de matières (166) par l'intermédiaire duquel le premier récipient étanche à l'air (70) recevant les matières est transféré, le trajet de transfert de cellules (165) et le trajet de transfert de matières (166) étant différents l'un de l'autre.

4. Système selon l'une quelconque des revendications 1 à 3,
dans lequel le trajet de transfert de cellules (165) et le trajet de transfert de matières (166) se croisent l'un l'autre de manière tridimensionnelle.

5. Système selon l'une quelconque des revendications 1 à 4,
dans lequel le dispositif de culture automatique (20, 30) est configuré pour effectuer automatiquement une sous-culture des cellules.

6. Système selon l'une quelconque des revendications 1 à 5,
dans lequel le premier récipient étanche à l'air (70) est transféré par la partie de transfert (60) dans un état dans lequel un second récipient étanche à l'air (75) qui reçoit les cellules est reçu à l'intérieur du premier récipient étanche à l'air (70).

7. Système selon la revendication 6,
dans lequel le premier récipient étanche à l'air (70) est transféré par la partie de transfert (60) dans un état dans lequel une pluralité de seconds récipients étanches à l'air (75) est reçue à l'intérieur du premier récipient étanche à l'air (70).

8. Système selon la revendication 6 ou 7,
dans lequel le dispositif de culture automatique (20, 30) est configuré pour recevoir le premier récipient étanche à l'air (70) qui reçoit le second récipient étanche à l'air (75) et qui est transféré par la partie de transfert (60), extraire le second récipient étanche à l'air (75) du premier récipient étanche à l'air (70), et mettre automatiquement en culture les cellules dans un état dans lequel les cellules sont reçues dans le second récipient étanche à l'air (75) extrait.

9. Système selon l'une quelconque des revendications 1 à 8, comprenant en outre :
un dispositif de stérilisation (1) configuré pour stériliser un intérieur du premier récipient étanche à l'air (70) qui reçoit les cellules.

10. Système selon la revendication 9,
dans lequel le dispositif de stérilisation (1) est configuré pour stériliser l'intérieur du premier récipient étanche à l'air (70) qui reçoit les cellules, en délivrant un gaz de stérilisation dans le premier récipient étanche à l'air (70).

11. Système selon l'une quelconque des revendications 1 à 10,
dans lequel le dispositif de culture automatique (20, 30) inclut une partie de rejet (18, 28, 38) configurée pour rejeter au moins un déchet liquide.

12. Système selon l'une quelconque des revendications 1 à 11,
dans lequel au moins une parmi une température, une humidité et une concentration de gaz à l'intérieur du premier récipient étanche à l'air (70) est réglable.

13. Système selon l'une quelconque des revendications 1 à 12, comprenant en outre :
une partie de stockage temporaire (5) configurée pour stocker temporairement le premier récipient étanche à l'air (70),
dans lequel au moins une parmi une température, une humidité et une concentration de gaz à l'intérieur de la partie de stockage temporaire (5) est réglable.

14. Système selon l'une des revendications 1 à 13,
dans lequel la partie de chargement (21, 31) inclut une partie de chargement de cellules (21a, 31a) configurée pour charger le premier récipient étanche à l'air (70) qui reçoit les cellules et une partie de chargement de matières (21b, 31b) configurée pour charger des matières.

15. Système selon l'une quelconque des revendications 1 à 14,
dans lequel le dispositif de culture automatique (20, 30) inclut une pluralité de dispositifs de culture automatique, le système comprend en outre une pluralité d'espaces de réception (100) configurés pour recevoir les dispositifs de culture automatique (20, 30), et les dispositifs de culture automatique (20, 30) sont respectivement reçus dans les espaces de réception (100) correspondants dans une correspondance bijective.

16. Procédé de culture automatique, comprenant :
transférer, par une partie de transfert (60), un premier récipient étanche à l'air (70) qui reçoit des cellules dans un état scellé ;
recevoir, par un dispositif de culture automatique (20, 30) ayant une partie de chargement (21, 31) agencée à l'intérieur d'une zone isolée, le premier récipient étanche à l'air (70) transféré par la partie de transfert (60) et extraire les cellules du premier récipient étanche à l'air (70) ; et
mettre en culture, par le dispositif de culture automatique (20, 30), les cellules extraites,
dans lequel la partie de transfert (60) inclut un plafond, un rail (65) agencé dans le plafond et une portion de maintien (61) configurée pour maintenir le premier récipient étanche à l'air (70) de telle sorte que le premier récipient étanche à l'air (70) soit suspendu vers le bas à partir du rail (65),
dans lequel la zone isolée est segmentée en une pluralité de zones par un organe d'isolement,
dans lequel la pluralité de zones inclut au moins deux zones segmentées par l'organe d'isolement, et
dans lequel le plafond inclut des plafonds des au moins deux zones, et le rail (65) s'étend entre les plafonds des au moins deux zones, et la partie de transfert (60) est configurée pour se déplacer le long du rail (65) et passer à travers l'organe d'isolement tout en maintenant le premier récipient étanche à l'air (70),
dans lequel le dispositif de culture automatique est configuré pour régler automatiquement au moins une parmi une température, une humidité et une concentration de gaz à l'intérieur du dispositif de culture automatique et pour effectuer automatiquement un remplacement d'un milieu de culture.
